# EUROPEAN PATENT SPECIFICATION

(11) **EP 2 950 804 B1**
(45) Date of publication and mention of the grant of the patent: **06.12.2017**
(21) Application number: 14709525.1
(22) Date of filing: 27.01.2014
(51) Int. Cl.: A61K 38/14, A61K 31/496, A61K 31/7056, A61K 33/38, A61P 31/04

(54) **ANTIBIOTIC PREPARATION AND USE THEREOF**
ANTIBIOTISCHE ZUSAMMENSETZUNG UND DEREN VERWENDUNG
PRÉPARATION ANTIBIOTIQUE ET SON UTILISATION

(30) Priority: 30.01.2013 CZ 20130062; 30.01.2013 CZ 201327385 U; 30.01.2013 CZ 201327386 U
(43) Date of publication of application: 09.12.2015
(73) Proprietor: Univerzita Palackého v Olomouci, 771 47 Olomouc (CZ)
(72) Inventor: PANACEK, Ales, 783 91 Unicov (CZ); KVITEK, Libor, 783 72 Velky Tynec (CZ); PRUCEK, Robert, 783 22 Olbramice (CZ); KOLAR, Milan, 779 00 Olomouc (CZ); ZBORIL, Radek, 779 00 Olomouc (CZ)
(74) Representative: Hartvichova, Katerina
(86) International application number: PCT/CZ2014/000010
(87) International publication number: WO 2014/117755

(56) References cited:
- RU-A- 2009 114 003
- US-A- 5 374 432
- GHOSH S ET AL: "Synthesis of silver nanoparticles using Dioscorea bulbifera tuber extract and evaluation of its synergistic potential in combination with antimicrobial agents", INTERNATIONAL JOURNAL OF NANOMEDICINE 2012 DOVE MEDICAL PRESS LTD. NZL, vol. 7, 2012, pages 483-496, XP002722984, ISSN: 1176-9114
- THANGAPANDIYAN S ET AL: "Chemically fabricated silver nanoparticles enhances the activity of antibiotics against selected human bacterial pathogens", INTERNATIONAL JOURNAL OF PHARMACEUTICAL SCIENCES AND RESEARCH 2012 SOCIETY OF PHARMACEUTICAL SCIENCES AND RESEARCH IND, vol. 3, no. 5, May 2012 (2012-05), pages 1415-1422, XP002722985, ISSN: 2320-5148
- CHAUDHARI P R ET AL: "Biosynthesis of silver nanoparticles using Saccharum officinarum and its antimicrobial activity", MICRO AND NANO LETTERS 2012 INSTITUTION OF ENGINEERING AND TECHNOLOGY GBR, vol. 7, no. 7, 2012, pages 646-650, XP002722986, ISSN: 1750-0443
- KINNEY E V ET AL: "Antibiotic-bonded PTFE vascular grafts: The effect of silver antibiotic on bioactivity following implantation", JOURNAL OF SURGICAL RESEARCH, ACADEMIC PRESS INC., SAN DIEGO, CA, US, vol. 50, no. 5, 1 May 1991 (1991-05-01), pages 430-435, XP026325936, ISSN: 0022-4804, DOI: 10.1016/0022-4804(91)90020-M [retrieved on 1991-05-01]
- PANACEK A ET AL: "Silver colloid nanoparticles: synthesis, characterization, and their antibacterial activity", JOURNAL OF PHYSICAL CHEMISTRY B ACS USA, vol. 110, no. 33, 24 August 2006 (2006-08-24), pages 16248-16253, XP002722987, ISSN: 1089-5647
- KVITEK L ET AL: "Initial study on the toxicity of silver nanoparticles (NPs) against Paramecium caudatum", JOURNAL OF PHYSICAL CHEMISTRY C AMERICAN CHEMICAL SOCIETY USA, vol. 113, no. 11, 19 March 2009 (2009-03-19), pages 4296-4300, XP002722988, ISSN: 1932-7447
- DURAN N. ET AL.: "Potential Use of Silver Nanoparticles on Pathogenic Bacteria, their Toxicity and Possible Mechanisms of Action", J. BRAZ. CHEM. SOC., vol. 21, no. 6, 2010, pages 949-959,
- WRIGHT J B ET AL: "Efficacy of topical silver against fungal burn wound pathogens", AMERICAN JOURNAL OF INFECTION CONTROL, C.V. MOSBY CO., ST. LOUIS, MO, US, vol. 27, no. 4, 1 August 1999 (1999-08-01) , pages 344-350, XP027397259, ISSN: 0196-6553 [retrieved on 1999-08-01]

## Description

### Field of Art

The present invention relates to antibiotic agents suitable in particular for use in human and veterinary medicine, health tools and materials, or in cosmetics in order to eliminate bacterial infections caused by bacterial microorganisms resistant to classical antibiotics.

### Background Art

Nowadays, systemic and local bacterial infections are treated with antibiotics, which are divided into several groups according to their chemical nature or the type of effect. Antibacterial activity of those agents varies, and minimum inhibition concentrations of the active compounds range from hundredths of mg/L up to units of mg/L, depending on the type of the target bacteria.

The treatment of systemic and local bacterial infections by means of commonly used antibiotics represents a significant medical problem caused by an increasing resistance of pathogenic bacteria towards antibiotic treatment. Bacterial resistance is not only a theory but it also has serious negative impacts in clinical medicine, including higher mortality and shorter survival time of patients with infections caused by multi-resistant bacteria. In certain cases it is necessary to use high antibiotic dosages which can be accompanied by undesirable side-effects, for example drug interaction and cytotoxicity. Factors influencing the formation and spreading of bacterial resistance against antibiotic treatment include antibiotic selective pressure, repeated administration of in particular wide-spectrum drugs, often in cases in which an antibiotic treatment is not necessary. There is a substantial risk of development of bacterial resistance against any newly developed specific antibiotic, caused by the ability of bacteria to quickly mutate against specific effects of antibiotics. For this reason there is nowadays a strong demand for new types of antibiotics, which would be effective at low concentrations, which would have none or minimum of undesirable side effects, and against which bacteria would not develop resistance.

If a newly developed agent should act effectively without being limited by bacterial resistance, it must act at several cell levels and not specifically as classical antibiotics do. Another possibility of elimination of bacterial resistance against antibiotics is an addition of a specific agent to the antibiotic, which disrupts the molecular biological processes in the bacterial cell responsible for the resistance. Such an example is the combination of β-lactam antibiotics with clavulanic acid which inhibits β-lactamases. Another example may be the use of aminoglycoside in combination with β-lactam antibiotics, which enables an easier antibiotic passage through the cell wall. The disadvantage of both agents (clavulanic acid and aminoglycoside) is their specific activity on one target, which can result in bacterial resistance even against these agents. A much more effective option to eliminate bacterial resistance might be an addition of a compound eliminating bacterial resistance through several molecular biological mechanisms to the antibiotic.

It is known that silver nanoparticles kill or inhibit the growth of pathogenic bacteria and yeast at concentrations from 0.1 mg/l to 5 mg/l (Panacek A. et al., J Phys Chem B, 110, 2006, 16248-16253; Kvitek L. et al., J Phys Chem C, 112, 2008, Panacek et al., Biomaterials, 30, 2009). It was also proved that colloid silver particles potentiate antibacterial effect of antibiotics. This property was however proved only for non-resistant bacteria (Li P. et al., Nanotechnology, 16, 2005; Shahverdi A.R. et al., Nanomedicine: Nanotechnology, Biology, and medicine, 3, 2007; Fayaz A.M et al., Nanomedicine: Nanotechnology, Biology, and medicine, 6, 2010). It was found that for bacteria resistant against some antibiotics, e.g. penicilin (*E. coli* DH5α (pPCRscript AMP SK⁺), *Pseudomonas aeruginosa, Enterobacter aerogenes* and methicillin-resistant *Staphylococcus aureus*) or ampicillin (*E. coli* DH5α (pPCRscript AMP SK⁺), *Pseudomonas aeruginosa*)*,* silver nanoparticles can show antibacterial activity, but solely when particles are functionalized on their surface directly by ampicillin, which binds to the nanoparticle surface via a sulphur atom contained in the molecule (Brown A. et al., Applied and Environmental Microbiology 78(8), 2012). This work however does not discuss the case when an antibiotic does not contain sulphur within its molecule, which is necessary to bind the antibiotic molecule on the silver nanoparticle surface. In patent applications WO 03/000303 and US2005/0192547, an antibiotic effect of an agent for catheter adjustment, which combines minocycline, rifampicin, norfloxacin or tobramicin antibiotics with various antiseptic agents, such as chlorhexidine, benzalkonium chloride, triclosan, bismuth salts or silver salts, was described. Tested pathogenic bacteria have not developed any resistance against the agent combining in particular minocycline (antibiotic) with triclosan (antiseptic agent) even after repeated exposure, and the agent showed the same antibiotic activity even upon repeated applications (20 repeated applications in total). Catheters treated with agents combining minocycline with chlorhexidine, minocycline with triclosan, rifampicin with chlorhexidine and rifampicin with triclosan showed antibiotic activity against resistant bacteria *S. epidermidis.* Such results are very interesting in the means of fighting against bacterial resistance, however, a fundamental problem of the use of such systems in practice is the high toxicity of the used organic antiseptic agents. This problem is even enhanced by the need to use relatively high antiseptic concentrations in order to show an effective antibiotic activity against the tested resistant bacterial strains. Antibacterial activity of a preparation combining antibiotics with silver or silver compounds against resistant bacteria was neither studied nor proved in this application. The only antibacterial activity studied was the effect of a preparation combining minocycline and two antiseptic agents, one of those being a silver compound Ag₂CO₃ or silver sulfadiazine at a considerably high concentration (0.75 % to 1 % (w/v)) and another antiseptic agent of the preparation being chlorhexidine diacetate or triclosan. Afterwards, the antibiotic activity of this preparation on a treated catheter was tested against non-resistant bacteria *S. epidermidis, P. aeruginosa* and *E. aerogenes.* Documents Ghosh et al., Int. J. Nanomed., vol. 7, 2012, 183-496; Thangapandiyan et al., Int. J. Pharmaceutical Sciences and Research, vol. 3, 2012, 1415-1422; Kinney et al., J. Surgical Research, vol. 50, 1991, 430-435; US 5374432 show the effect of silver or combination of silver with antibiotics towards sensitive bacteria.

Documents Chaudhari et al., Micro and Nano Letters, vol. 7, 2012, 646-650; Duran et al., J. Braz. Chem. Soc., vol. 21, 2010, 949-959 show the use of silver against sensitive and resistant bacteria in amounts exceeding minimum inhibitory concentrations, therefore in these documents, silver is the used as antibacterial agent. Document Kvitek et al., J. Phys. Chem. C, vol. 113, 2009, 4296-4300 shows the inhibitory effect of silver against an eucaryotic organism *Paramecium caudatum.*

Therefore it can be concluded that up to now no effective combination of an antibiotic with another agent, which would be able to generally restore antibiotic activity of the antibiotic against resistant bacterial strains, preferably at low concentrations corresponding to serum concentrations of antibiotics in human body, was found.

### Disclosure of the Invention

The object of the present invention is a preparation containing an antibiotic and silver in the form selected from nanoparticles and silver compound, for use in the treatment of infections caused by bacteria resistant against the antibiotic.

A further object of the invention is use of the combination of an antibiotic and silver in the form selected from nanoparticles and silver compound for the preparation of a drug for the treatment of infections caused by antibiotic-resistant bacteria.

The present invention further includes a method of treatment of infections caused by antibiotic-resistant bacteria, comprising the step of administration of the combination of an antibiotic and silver in the form selected from nanoparticles and silver compound to a patient in need of such treatment.

The present invention includes also a method and a form of application of the preparation or the drug for the treatment of infections caused by antibiotic-resistant bacteria, including topical administration in the form of plaster, powder, ointment, gel, solution or dispersion, oral administration in the form of tablets, solution or dispersion, intravenous administration in the form of solution or dispersion, and administration via inhalation in the form of aerosol, of the combination of an antibiotic and silver in the form of nano- or microparticles or in the form of a silver compound to a patient.

The present invention includes also use of the preparation containing an antibiotic and silver in the form of nanoparticles or in the form of a silver compound for the treatment of surfaces, in particular medical, cosmetic, laboratory and other surfaces, devices and instruments, and/or for the treatment of materials, in particular medical, cosmetic, laboratory materials, against antibiotic-resistant bacteria.

The present invention further includes an antibiotic preparation containing a mixture of an antibiotic and silver nanoparticles, preferably without chemical bonds between the antibiotic and silver.

An antibiotic is any antibiotic with the exclusion of β-lactam antibiotics.

The invention is based on a surprising restoration of the antibiotic activity of antibiotics using metallic silver in the form of nanoparticles or in the form of a silver compound with no need for chemical bonding of antibiotic molecules on the surface of silver nanoparticles via sulfur heteroatoms present in the antibiotic molecules, as it is in the case of penicillin-type antibiotics (β-lactam antibiotics). The antibiotic in combination with silver or silver compound shows again an antibiotic effect against those resistant bacteria, against which the same antibiotic free of silver has no or only a partial antibiotic effect. Therefore, restoration of a full sensitivity of bacteria towards the antibiotic takes place, if the antibiotic is combined with silver or silver compounds. At the same time, such combination of the antibiotic and silver extends its efficiency upon a whole range of bacterial strains, against which the antibiotic was initially ineffective (for example the extension of the antibiotic activity of vankomycin against gram-negative bacteria). Such combination of an antibiotic and silver achieves the properties of broad-spectrum antibiotics even if the antibiotic alone does not belong among broad-spectrum antibiotics.

A preparation containing antibiotic and silver or silver compounds can be in the form of dispersion systems, such as aqueous and alcoholic solutions, aqueous and alcoholic dispersions, emulsions, gels, ointments or powders destined to inhibit and/or kill bacteria.

The advantage of the use of silver according to the present invention in order to restore antibiotic activity of antibiotics, in comparison with the prior art, is the fact that silver eliminates bacterial resistance towards antibiotics by several different molecular-biological mechanisms. Hence, it eliminates the possibility of bacterial resistance towards the agent restoring antibiotic properties of the antibiotics. Further, it is possible to arbitrarily combine silver or silver compounds with any antibiotic independently on its chemical structure or mechanism of activity.

Another advantage of silver or silver compound addition to antibiotics is the fact that even at very low silver concentrations in the final preparation, leading to serum concentration values of silver in the order of mg/L, the increase of antibiotic activity of antibiotics is multifold, and therefore the antibiotic containing silver or silver compounds can be used at lower concentrations than the antibiotic alone. This accompanying effect can lead towards an important decrease of undesirable side effects on animals or humans, to whom the preparation has been administered. Moreover, it was proved herein that, in comparison to the results published earlier, the concentration of antibiotics can be lowered to values smaller than the therapeutic dose of the antibiotic alone against non-resistant bacterial strains. This leads to an important decrease of undesirable side effects compared to the application of high dosages of antibiotics due to infections caused by resistant bacterial strains.

A suitable form of silver is silver in the form of nano- or microparticles and/or in the form of an inorganic or organic silver compound, e.g., salts, oxides or complexes, such as AgNO₃, CH₃COOAg, AgCl, AgBr, AgI, AgOH, Ag₂O, Ag₂CO₃, Ag₂SO₄, Ag₃PO₄, AgClO₄ etc.

Silver in the form of nanoparticles is applied at concentrations of silver in the preparation from 1 mg/L to 20 mg/L for silver particle size from 1 nm to 100 nm, in order to restore the antibiotic activity of antibiotics. The mentioned higher ranges of the concentrations are suitable mainly for topical applications of the preparations containing silver or silver compounds, the lower ranges of concentrations are more suitable for other ways of administration.

When silver nanoparticles and microparticles are used, it is preferable to use a stabilizing agent in the preparation, which ensures the aggregate and sedimentation stability of the present silver particles. Any compound exhibiting a stabilizing effect against aggregation and sedimentation of silver particles can serve as the stabilizing agent. Examples of stabilizing agents are synthetic polymer stabilizers (polyethylene glycols, polyvinylpyrrolidones, polyvinylalcohols, polyacrylates, fatty acids etc.), as well as natural polymer stabilizers (proteins, polysaccharides) or ionic and non-ionic surfactants.

Organic and inorganic silver compounds can be used at concentrations from 0.01 mg/L to 10 mg/L of silver. For soluble silver compounds it is preferable to use concentrations of silver from 0.01 mg/L to 5 mg/L, and for less soluble silver compounds it is preferable to use concentrations of silver from 0.1 mg/L to 10 mg/L.

The concentration of antibiotics in combination with silver or silver compounds for the applications against resistant bacteria can remain in the conventional range which is used for the treatment of an infection caused by non-resistant bacteria with the antibiotic alone. Alternatively, such combination enables to even lower the antibiotic concentration.

The antibiotics are mainly selected from the antibiotics with the mechanism of action based on the inhibition of the synthesis of substances of the bacterial cell wall (for example glycopeptide antibiotics, e.g. vancomycin), antibiotics inhibiting the bacterial protein synthesis (for example lincosamide antibiotics, e.g. clindamycin, aminoglycoside antibiotics, e.g. gentamicin), antibiotics inhibiting the synthesis of bacterial nucleic acids (fluoroquinolone antibiotics, e.g. ciprofloxacin), antibiotics inhibiting bacterial metabolism and antibiotics disrupting bacterial cytoplasmic membrane.

Silver containing antibiotics can therefore be used in human or veterinary medicine especially in the treatment of infections caused by highly resistant pathogenic bacteria, where the treatment with classical antibiotics fails because of bacterial resistance. Such bacteria include for example strains *Staphylococcus aureus* MRSA or clindamycin resistant, *Enterococcus faecium* VRE strains, *Klebsiella pneumoniae* ESBL-positive strains, *Escherichia coli* ESBL-positive strains etc. Moreover, considering the very high antibiotic activity of the silver containing antibiotic, it is possible to administer the preparation at manifold lower concentrations than when the antibiotic is being administered separately, free of silver. The risk of undesirable side effects related to, e.g., the toxicity of the used antibiotics is therefore also lowered.

### Examples

### Example 1

### Restoration of antibiotic activity of ciprofloxacin against resistant bacteria Escherichia coli (ESBL-positive strain) using silver nanoparticles

Antibiotic activities of ciprofloxacin and of ciprofloxacin with the addition of silver nanoparticles were tested against ciprofloxacin-resistant bacteria *Escherichia coli* (ESBL-positive strain). The breakpoint value of ciprofloxacin against *Escherichia coli* (ESBL-positive strain) is, according to the database "The European Committee on Antimicrobial Susceptibility Testing" - further only EUCAST, 1 mg/L. Ciprofloxacin was tested in concentration intervals from 0.05 mg/L to 16 mg/L. In order to restore ciprofloxacin activity, silver nanoparticles of the size of 25 nm and of silver concentration of 3 mg/L and 5 mg/L were used. Silver concentrations were chosen lower than it is the value of minimal inhibition concentration of silver nanoparticles, which was 7.5 mg/L of silver, when used against *Escherichia coli* (ESBL-positive strain). Ciprofloxacin antibiotic activity was determined by a standard microdilution method using a microtitration plate. Bacteria *Escherichia coli* (ESBL-positive strain) was cultivated in MH nutrient broth for 24 hours at 37 °C. The growth (+) and growth inhibition (-) of bacteria were observed.

| Tested material | Concentration (mg/L) | growth |
|---|---|---|
| ciprofloxacin | 2 | + |
| | 8 | + |
| | 16 | + |
| ciprofloxacin + NanoAg | 2 + 3 | - |
| | 0.1 + 5 | - |
| | 0.05 + 5 | - |

| | | |
|---|---|---|
| + bacteria grows; - bacteria does not grow | | |

### Example 2

### Restoration of antibiotic activity of ciprofloxacin against resistant bacteria KPC-Klebsiella pneumoniae using silver nanoparticles

Antibiotic activities of ciprofloxacin and of ciprofloxacin with the addition of silver nanoparticles were tested against ciprofloxacin-resistant bacteria KPC-*Klebsiella pneumoniae.* The breakpoint value of ciprofloxacin against *KPC-Klebsiella pneumoniae* is, according to the database EUCAST, 1 mg/L. Ciprofloxacin was tested in concentration intervals from 0.05 mg/L to 16 mg/L. In order to restore ciprofloxacin activity, silver nanoparticles of the size of 25 nm and of silver concentration of 3 mg/L and 5 mg/L were used. Silver concentrations were chosen lower than it is the value of minimal inhibition concentration of silver nanoparticles, which was 7.5 mg/L of silver, when used against KPC*-Klebsiella pneumoniae.* Ciprofloxacin antibiotic activity was determined by a standard microdilution method using a microtitration plate. Bacteria KPC-*Klebsiella pneumoniae* was cultivated in MH nutrient broth for 24 hours at 37 °C. The growth (+) and growth inhibition (-) of bacteria were observed.

| Tested material | Concentration (mg/L) | growth |
|---|---|---|
| ciprofloxacin | 2 | + |
| | 8 | + |
| | 16 | + |
| ciprofloxacin + NanoAg | 2 + 3 | - |
| | 0.1 + 5 | - |
| | 0.05 + 5 | - |

| | | |
|---|---|---|
| + bacteria grows; - bacteria does not grow | | |

### Example 3

### Restoration of antibiotic activity of vancomycin against multiresistant bacteria Enterococcus faecium (VRE) using silver nanoparticles

Antibiotic activities of vancomycin and of vancomycin with the addition of silver nanoparticles were tested against vancomycin -resistant bacteria *Enterococcus faecium* (VRE). The breakpoint value of vancomycin against *Enterococcus faecium* (VRE) is, according to the database EUCAST, 4 mg/L. Vancomycin was tested in concentration intervals from 0.1 mg/L to 16 mg/L. In order to restore vancomycin activity, silver nanoparticles of the size of 25 nm and of silver concentration of 3 mg/L and 5 mg/L were used. Silver concentrations were chosen lower than it is the value of minimal inhibition concentration of silver nanoparticles, which was 7.5 mg/L of silver, when used against *Enterococcus faecium* (VRE). Vancomycin antibiotic activity was determined by a standard microdilution method using a microtitration plate. Bacteria *Enterococcus faecium* (VRE) was cultivated in MH nutrient broth for 24 hours at 37 °C. The growth (+) and growth inhibition (-) of bacteria were observed.

| Tested material | Concentration (mg/L) | growth |
|---|---|---|
| vancomycin | 4 | + |
| | 8 | + |
| | 16 | + |
| vancomycin + NanoAg | 1 + 3 | - |
| | 0.5 + 5 | - |
| | 0.1 + 5 | - |

| | | |
|---|---|---|
| + bacteria grows; - bacteria does not grow | | |

### Example 4

### Restoration of antibiotic activity of gentamicin against gentamicin-resistant bacteria strain Klebsiella pneumoniae (ESBL-positive strain) using silver nanoparticles

Antibiotic activities of gentamicin and of gentamicin with the addition of silver nanoparticles were tested against gentamicin-resistant bacteria *Klebsiella pneumoniae* (ESBL-positive strain). The breakpoint value of gentamicin against *Klebsiella pneumoniae* (ESBL-positive strain) is, according to the database EUCAST, 4 mg/L. Gentamicin was tested in concentration intervals from 0.1 mg/L to 16 mg/L. In order to restore gentamicin activity, silver nanoparticles of the size of 25 nm and of silver concentration of 3 mg/L and 5 mg/L were used. Silver concentrations were chosen lower than it is the value of minimal inhibition concentration of silver nanoparticles, which was 7.5 mg/L of silver, when used against *Klebsiella pneumoniae* (ESBL-positive strain). Gentamicin antibiotic activity was determined by a standard microdilution method using a microtitration plate. Bacteria *Klebsiella pneumoniae* (ESBL-positive strain) was cultivated in MH nutrient broth for 24 hours at 37 °C. The growth (+) and growth inhibition (-) of bacteria were observed.

| Tested material | Concentration (mg/L) | growth |
|---|---|---|
| gentamicin | 4 | + |
| | 8 | + |
| | 16 | + |
| gentamicin + | 1 + 3 | |
| NanoAg | 0.5 + 5 | - |
| | 0.1 + 5 | - |

| | | |
|---|---|---|
| + bacteria grows; - bacteria does not grow | | |

### Example 5

### Restoration of antibiotic activity of ciprofloxacin against ciprofloxacin-resistant bacteria Escherichia coli (ESBL-positive strain) using AgNO₃

Antibiotic activities of ciprofloxacin and of ciprofloxacin with the addition of AgNO₃ were tested against ciprofloxacin-resistant bacteria *Escherichia coli* (ESBL-positive strain). The breakpoint value of ciprofloxacin against *Escherichia coli* (ESBL-positive strain) is, according to the database EUCAST, 1 mg/L. Ciprofloxacin was tested in concentration intervals from 0.05 mg/L to 16 mg/L. In order to restore ciprofloxacin activity, AgNO₃ of silver concentration of 0.5 mg/L and 1 mg/L were used. Silver concentrations were chosen lower than it is the value of minimal inhibition concentration of AgNO₃, which was 3 mg/L of silver, when used against *Escherichia coli* (ESBL-positive strain). Ciprofloxacin antibiotic activity was determined by a standard microdilution method using a microtitration plate. Bacteria *Escherichia coli* (ESBL-positive strain) was cultivated in MH nutrient broth for 24 hours at 37 °C. The growth (+) and growth inhibition (-) of bacteria were observed.

| Tested material | Concentration (mg/L) | growth |
|---|---|---|
| ciprofloxacin | 2 | + |
| | 8 | + |
| | 16 | + |
| ciprofloxacin + AgNO₃ | 2 + 0.5 | - |
| | 0.1 + 1 | - |
| | 0.05 + 1 | - |

| | | |
|---|---|---|
| + bacteria grows; - bacteria does not grow | | |

### Example 6

### Restoration of antibiotic activity of ciprofloxacin against resistant bacteria KPC-Klebsiella pneumoniae using Ag₂O

Antibiotic activities of ciprofloxacin and of ciprofloxacin with the addition of Ag₂O were tested against ciprofloxacin-resistant bacteria KPC-*Klebsiella pneumoniae.* The breakpoint value of ciprofloxacin against KPC-*Klebsiella pneumoniae* is, according to the database EUCAST, 1 mg/L. Ciprofloxacin was tested in concentration interval from 0.05 mg/L to 16 mg/L. In order to restore ciprofloxacin activity, Ag₂O of silver concentration of 1 mg/L and 3 mg/L were used. Silver concentrations were chosen lower than it is the value of minimal inhibition concentration of Ag₂O, which was 7.5 mg/L of silver, when used against KPC-*Klebsiella pneumoniae.* Ciprofloxacin antibiotic activity was determined by a standard microdilution method using a microtitration plate. Bacteria KPC-*Klebsiella pneumoniae* was cultivated in MH nutrient broth for 24 hours at 37 °C. The growth (+) and growth inhibition (-) of bacteria were observed.

| Tested material | Concentration (mg/L) | growth |
|---|---|---|
| ciprofloxacin | 2 | + |
| | 8 | + |
| | 16 | + |
| ciprofloxacin + Ag₂O | 2 + 1 | - |
| | 0.1 + 3 | - |
| | 0.05 + 3 | - |

| | | |
|---|---|---|
| + bacteria grows; - bacteria does not grow | | |

### Example 7

### Restoration of antibiotic activity of clindamycin in dermatological emulsion (Dalacin T 1 %) against bacteria Staphylococcus aureus using silver nanoparticles

Antibiotic activities of a dermatological emulsion containing clindamycin in a concentration of 10 g/L and of a dermatological emulsion containing clindamycin in a concentration of 10 g/L with the addition of silver nanoparticles were tested against clindamycin-resistant bacteria *Staphylococcus aureus.* The breakpoint value of clindamycin against *Staphylococcus aureus* is, according to the database EUCAST, 0.5 mg/L. Clindamycin was tested in a non-changed concentration of 10 g/L in the preparation Dalacin T. In order to restore clindamycin activity, silver nanoparticles of the size of 25 nm and of silver concentration of 100 mg/L and 50 mg/L in the preparation were used. Silver nanoparticles were added into the preparation Dalacin T in a form of a concentrated dispersion and subsequently homogenized. Clindamycin antibiotic activity was determined by a disc diffusion test in a Petri dish. Bacteria *Staphylococcus aureus* was cultivated in agar for 24 hours at 37 °C. The inhibition zone in the vicinity of the applied sample was observed.

| Tested material | concentration (g/L) | Inhibition zone (mm) |
|---|---|---|
| clindamycin | 10 | 0 |
| clindamycin + NanoAg | 10 + 0.1 | 11 |
| clindamycin + NanoAg | 10 + 0.05 | 9 |

## Claims

1. A preparation containing an antibiotic, with the exclusion of β-lactam antibiotics, and silver in the form selected from nanoparticles at concentrations 1 mg/L to 20 mg/L for silver particle size from 1 nm to 100 nm, and silver compound at concentrations of silver from 0.01 mg/L to 10 mg/L, for use in the treatment of infections caused by bacteria resistant against the antibiotic.

2. Use of a preparation containing an antibiotic, with the exclusion of β-lactam antibiotics, and silver in the form selected from nanoparticles at concentrations 1 mg/L to 20 mg/L for silver particle size from 1 nm to 100 nm, and silver compound at concentrations of silver from 0.01 mg/L to 10 mg/L, for the treatment of surfaces and/or for the treatment of materials against antibiotic-resistant bacteria.

3. The preparation for use according to claim 1 or use according to claim 2, wherein the preparation containing the antibiotic and silver or silver compounds is in the form selected from the group comprising aqueous solutions, aqueous dispersions, emulsions, gels, ointments or powders.

4. The preparation for use or use according to any preceding claim, wherein the silver compounds are selected from the group comprising AgNO₃, CH₃COOAg, AgCl, AgBr, AgI, AgOH, Ag₂O, Ag₂CO₃, Ag₂SO₄, Ag₃PO₄, AgClO₄.

5. The preparation for use or use according to any of claims 1 to 3, wherein the nanoparticles are present in the preparation in concentration from 1 mg/L to 10 mg/L of silver, and have the size from 1 nm to 100 nm.

6. The preparation for use or use according to any preceding claim, wherein the preparation further contains at least one stabilizing agent, preferably selected from synthetic polymer stabilizers, natural polymer stabilizers and surfactants.

## Patentansprüche

1. Präparat, das ein Antibiotikum, unter Ausschluss von β-Lactam-Antibiotika, und Silber in der Form ausgewählt aus Nanopartikeln in Konzentrationen von 1 mg/L bis 20 mg/L für Silberpartikelgröße von 1 nm bis 100 nm und Silberverbindung bei Konzentrationen von Silber von 0,01 mg/L bis 10 mg/L enthält, zur Verwendung bei der Behandlung von Infektionen, die durch Bakterien verursacht wurden, die gegen das Antibiotikum resistent sind.

2. Verwendung eines Präparates, das ein Antibiotikum, unter Ausschluss von β-lactam-Antibiotika, und Silber in der Form ausgewählt aus Nanopartikeln bei Konzentrationen von 1 mg/L bis 20 mg/L für Silberpartikelgröße von 1 nm bis 100 nm und Silber-Verbindung bei Silberkonzentrationen von 0,01 mg/L bis 10 mg/L enthält, zur Behandlung von Oberflächen und/oder zur Behandlung von Materialen gegen antibiotika-resistenten Bakterien.

3. Präparat zur Verwendung nach Anspruch 1 oder Verwendung nach Anspruch 2, wobei das Präparat, das das Antibiotikum und Silber oder Silberverbindungen enthält, in der Form ausgewählt aus der Gruppe bestehend aus wässrigen Lösungen, wässrigen Dispersionen, Emulsionen, Gelen, Salben oder Pulvern ist.

4. Präparat zur Verwendung oder Verwendung nach einem der vorhergehenden Ansprüche, wobei die Silberverbindungen ausgewählt sind aus der Gruppe umfassend AgNO₃, CH₃COOAg, AgCl, AgBr, AgI, AgOH, Ag₂O, Ag₂CO₃, Ag₂SO₄, Ag₃PO₄, AgClO₄.

5. Präparat zur Verwendung oder Verwendung nach einem der Ansprüche 1 bis 3, wobei die Nanopartikel in das Präparat in einer Konzentration von 1 mg/L bis 10 mg/L Silber vorliegen und die Größe von 1 nm bis 100 nm aufweisen.

6. Präparat zur Verwendung oder Verwendung nach einem der vorhergehenden Ansprüche, wobei das Präparat ferner mindestens ein Stabilisierungsmittel enthält, vorzugsweise ausgewählt aus synthetischen Polymerstabilisatoren, natürlichen Polymerstabilisatoren und Tensiden.

## Revendications

1. Une préparation contenant un antibiotique, à l'exclusion des antibiotiques ß-lactame, et
de l'argent sous la forme choisie parmi les nanoparticules à des concentrations de 1 mg/L à 20 mg/L pour une taille de particule d'argent de 1 nm à 100 nm, et un composé d'argent à des concentrations d'argent de 0,01 mg/L à 10 mg/L,
pour une utilisation dans le traitement d'infections causées par des bactéries bactéries résistantes au cet antibiotique.

2. Utilisation d'une préparation contenant un antibiotique, à l'exclusion des antibiotiques β-lactame, et de l'argent sous la forme choisie parmi les nanoparticules à des concentrations de 1 mg/L à 20 mg/L pour une taille de particule d'argent de 1 nm à 100 nm et composé d'argent à des concentrations d'argent de 0,01 mg/L à 10 mg/L, pour le traitement des surfaces et/ou pour le traitement des matériaux contre les bactéries résistant aux antibiotiques.

3. Préparation pour l'utilisation selon la revendication 1 ou l'utilisation selon la revendication 2, où la préparation contenant l'antibiotique et argent ou le composé d'argent est sous la forme choisie dans le groupe comprenant des solutions aqueuses, des dispersions aqueuses, des emulsions, des gels, des pommades ou des poudres.

4. Préparation pour l'utilisation ou l'utilisation selon l'une quelconque des revendications précédentes, où les composés d'argent sont choisis dans le groupe comprenant AgNO₃, CH₃COOAg, AgCl, AgBr, AgI, AgOH, Ag₂O, Ag₂CO₃, Ag₂SO₄, Ag₃PO₄, AgClO₄.

5. Préparation pour l'utilisation ou l'utilisation selon l'une quelconque des revendications 1 à 3, où les nanoparticules sont présentes dans la préparation en concentration de 1 mg/L à 10 mg/L d'argent et ont la taille de 1 nm à 100 nm.

6. Préparation pour l'utilisation ou l'utilisation selon l'une quelconque des revendications précédentes, où la préparation contient en outre au moins un agent stabilisant, de préférence choisi parmi des stabilisants polymères synthétiques, des stabilisants polymères naturels et des tensioactifs.
